# EUROPEAN PATENT SPECIFICATION

(11) **EP 1 225 836 B1**
(45) Date of publication and mention of the grant of the patent: **10.12.2008**
(21) Application number: 00967148.8
(22) Date of filing: 29.09.2000
(51) Int. Cl.: A61B 17/12

(54) **FILAMENTOUS EMBOLIC DEVICE WITH EXPANSIBLE ELEMENTS**
FADENFÖRMIGE EMBOLIEVORRICHTUNG MIT AUSDEHNBAREN ELEMENTEN
DISPOSITIF D'EMBOLISATION FILAMENTEUX A ELEMENTS EXPANSIBLES

(30) Priority: 04.10.1999 US 410970; 04.04.2000 US 542145
(43) Date of publication of application: 31.07.2002
(60) Divisional application: 08015975.9
(73) Proprietor: Microvention, Inc., Aliso Viejo, CA 92656 (US)
(72) Inventor: GREENE, George, R., Jr., Costa Mesa, CA 92626 (US); ROSENBLUTH, Robert, F., Laguna Niguel, CA 92677 (US); COX, Brian, J., Laguna Niguel, CA 92677 (US)
(74) Representative: Nielsen, Henrik Sten
(86) International application number: PCT/US2000/026926
(87) International publication number: WO 2001/028434

(56) References cited:
- US-A- 5 582 619
- US-A- 5 690 671
- US-A- 5 718 711
- US-A- 5 750 585
- US-A- 5 766 219
- US-A- 5 823 198

## Description

### BACKGROUND OF THE INVENTION

The present invention relates to the field of methods and devices for the embolization of vascular aneurysms and similar vascular abnormalities. More specifically, the present invention relates to an embolic device that is inserted into a vascular site such as an aneurysm to create an embolism therein and a method for embolizing a vascular site using the device.

The embolization of blood vessels is desired in a number of clinical situations. For example, vascular embolization has been used to control vascular bleeding, to occlude the blood supply to tumors, and to occlude vascular aneurysms, particularly intracranial aneurysms. In recent years, vascular embolization for the treatment of aneurysms has received much attention. Several different treatment modalities have been employed in the prior art. U.S. Patent No. 4,819,637 - Dormandy, Jr. et al., for example, describes a vascular embolization system that employs a detachable balloon delivered to the aneurysm site by an intravascular catheter. The balloon is carried into the aneurysm at the tip of the catheter, and it is inflated inside the aneurysm with a solidifying fluid (typically a polymerizable resin or gel) to occlude the aneurysm. The balloon is then detached from the catheter by gentle traction on the catheter. While the balloon-type embolization device can provide an effective occlusion of many types of aneurysms, it is difficult to retrieve or move after the solidifying fluid sets, and it is difficult to visualize unless it is filled with a contrast material. Furthermore, there are risks of balloon rupture during inflation and of premature detachment of the balloon from the catheter.

Another approach is the direct injection of a liquid polymer embolic agent into the vascular site to be occluded. One type of liquid polymer used in the direct injection technique is a rapidly polymerizing liquid, such as a cyanoacrylate resin, particularly isobutyl cyanoacrylate, that is delivered to the target site as a liquid, and then is polymerized *in situ*. Alternatively, a liquid polymer that is precipitated at the target site from a carrier solution has been used. An example of this type of embolic agent is a cellulose acetate polymer mixed with bismuth trioxide and dissolved in dimethyl sulfoxide (DMSO). Another type is ethylene vinyl alcohol dissolved in DMSO. On contact with blood, the DMSO diffuses out, and the polymer precipitates out and rapidly hardens into an embolic mass that conforms to the shape of the aneurysm. Other examples of materials used in this "direct injection" method are disclosed in the following U.S. Patents: 4,551,132 - Pásztor et al.; 4,795,741 - Leshchiner et al.; 5,525,334 - Ito et al.; and 5,580,568 - Greff et al.

The direct injection of liquid polymer embolic agents has proven difficult in practice. For example, migration of the polymeric material from the aneurysm and into the adjacent blood vessel has presented a problem. In addition, visualization of the embolization material requires that a contrasting agent be mixed with it, and selecting embolization materials and contrasting agents that are mutually compatible may result in performance compromises that are less than optimal. Furthermore, precise control of the deployment of the polymeric embolization material is difficult, leading to the risk of improper placement and/or premature solidification of the material. Moreover, once the embolization material is deployed and solidified, it is difficult to move or retrieve.

Another approach that has shown promise is the use of thrombogenic microcoils. These microcoils may be made of a biocompatible metal alloy (typically platinum and tungsten) or a suitable polymer. If made of metal, the coil may be provided with Dacron fibers to increase thrombogenicity. The coil is deployed through a microcatheter to the vascular site. Examples of microcoils are disclosed in the following U.S. patents: 4,994,069 - Ritchart et al.; 5,133,731 - Butler et al.; 5,226,911 - Chee et al.; 5,312,415 - Palermo; 5,382,259 - Phelps et al.; 5,382,260 - Dormandy, Jr. et al.; 5,476,472 - Dormandy, Jr. et al.; 5,578,074 - Mirigian; 5,582,619 - Ken; 5,624,461 - Mariant; 5,645,558 - Horton; 5,658,308 - Snyder; and 5,718,711 - Berenstein et al.

The microcoil approach has met with some success in treating small aneurysms with narrow necks, but the coil must be tightly packed into the aneurysm to avoid shifting that can lead to recanalization. Microcoils have been less successful in the treatment of larger aneurysms, specially those with relatively wide necks. A disadvantage of microcoils that they are not easily retrievable; if a coil migrates out of the aneurysm, a second procedure to retrieve it and move it back into place is necessary. Furthermore, complete packing of an aneurysm using microcoils can be difficult to achieve in practice.

A specific type of microcoil that has achieved a measure of success is the Guglielmi Detachable Coil ("GDC"), described in U.S. Patent No. 5,122,136 - Guglielmi et al. The GDC employs a platinum wire coil fixed to a stainless steel delivery wire by a solder connection. After the coil is placed inside an aneurysm, an electrical current is applied to the delivery wire, which heats sufficiently to melt the solder junction, thereby detaching the coil from the delivery wire. The application of the current also creates a positive electrical charge on the coil, which attracts negatively-charged blood cells, platelets, and fibrinogen, thereby increasing the thrombogenicity of the coil. Several coils of different diameters and lengths can be packed into an aneurysm until the aneurysm is completely filled. The coils thus create and hold a thrombus within the aneurysm, inhibiting its displacement and its fragmentation.

The advantages of the GDC procedure are the ability to withdraw and relocate the coil if it migrates from its desired location, and the enhanced ability to promote the formation of a stable thrombus within the aneurysm. Nevertheless, as in conventional microcoil techniques, the successful use of the GDC procedure has been substantially limited to small aneurysms with narrow necks.

Still another approach to the embolization of an abnormal vascular site is the injection into the site of a biocompatible hydrogel, such as poly (2-hydroxyethyl methacrylate) ("pHEMA" or "PHEMA"); or a polyvinyl alcohol foam ("PAF"). See, e.g., Horák et al., "Hydrogels in Endovascular Embolization. II. Clinical Use of Spherical Particles", Biomaterials, Vol. 7, pp. 467-470 (Nov., 1986); Rao et al., "Hydrolysed Microspheres from Cross-Linked Polymethyl Methacrylate", J. Neuroradiol., Vol. 18, pp. 61-69 (1991); Latchaw et al., "Polyvinyl Foam Embolization of Vascular and Neoplastic Lesions of the Head, Neck, and Spine", Radiology, Vol. 131, pp. 669-679 (June, 1979). These materials are delivered as microparticles in a carrier fluid that is injected into the vascular site, a process that has proven difficult to control.

A further development has been the formulation of the hydrogel materials into a preformed implant or plug that is installed in the vascular site by means such as a microcatheter. See, e.g., U.S. Patent No. 5,258,042 - Mehta. These types of plugs or implants are primarily designed for obstructing blood flow through a tubular vessel or the neck of an aneurysm, and they are not easily adapted for precise implantation within a sac-shaped vascular structure, such as an aneurysm, so as to fill substantially the entire volume of the structure.

U.S. Patent No. 5,823,198 - Jones et al. discloses an expansible PVA foam plug that is delivered to the interior of an aneurysm at the end of a guidewire. The plug comprises a plurality of pellets or particles that expand into an open-celled structure upon exposure to the fluids within the aneurysm so as to embolize the aneurysm. The pellets are coated with a blood-soluble restraining agent to maintain them in a compressed state and attached to the guidewire until delivered to the aneurysm. Because there is no mechanical connection between the pellets and the guidewire (other than the relatively weak temporary bond provided by the restraining agent), however, premature release and migration of some of the pellets remains a possibility.

U.S. patent No. 5766219 discloses an occlusive device for insertion into a body cavity. The device comprises a number of embolizing elements non-releasably carried on a filamentous carrier at spaced intervals along the carrier. The material of the embolizing elements is suggested to possibly being polyvinyl alcohol foam, which is known to be hydrophilic.

There has thus been a long-felt, but as yet unsatisfied need for an aneurysm treatment device and method that can substantially fill aneurysms of a large range of sizes, configurations, and neck widths with a thrombogenic medium with a minimal risk of inadvertent aneurysm rupture or blood vessel wall damage. There has been a further need for such a method and device that also allow for the precise locational deployment of the medium, while also minimizing the potential for migration away from the target location. In addition, a method and device meeting these criteria should also be relatively easy to use in a clinical setting. Such ease of use, for example, should preferably include a provision for good visualization of the device during and after deployment in an aneurysm.

### SUMMARY OF THE INVENTION

Broadly, an embolization device, according to the present invention, comprises one or more expansible, hydrophilic embolizing elements non-releasably carried on a filamentous elastic memory carrier at spaced intervals along the length of the carrier, said one or more embolizing elements being cylindrical micropellets. In a preferred embodiment, the carrier is a suitable length of very thin, highly flexible filament of nickel/titanium alloy. The embolizing elements are separated from each other on the carrier by radiopaque spacers in the form of highly flexible microcoils made of platinum or platinum/tungsten alloy, as in the thrombogenic microcoils of the prior art, as described above.

In a preferred embodiment, the embolizing elements are made of a hydrophilic, macroporous, polymeric, hydrogel foam material, in particular a swellable foam matrix formed as a macroporous solid comprising a foam stabilizing agent and a polymer or copolymer of a free radical polymerizable hydrophilic olefin monomer cross-linked with up to about 10% by weight of a multiolefin-functional cross-linking agent. Such a material is described in U.S. Patent No. 5,750,585 - Park et al., the disclosure of which is incorporated herein by reference. The material may be modified, or provided with additives, to make the implant visible by conventional imaging technique.

The present description describes the use of the embolizing device in a method for embolizing a vascular site, comprising, in the preferred embodiment the steps of: (a) passing a microcatheter intravascularly so that its distal end is introduced into a target vascular site; (b) passing a vaso-occlusive device through the microcatheter into the target vascular site so that the vaso-occlusive device assumes a three-dimensional configuration that fills a portion of the volume of the target vascular site; (c) providing a vascular embolization device comprising at least one expansible embolizing element non-releasably connected to a filamentous carrier; (d) passing the embolization device through the microcatheter so that it emerges from the distal end of the microcatheter into the target vascular site; and (e) expanding the embolizing element or elements *in situ* substantially to fill the remaining volume of the target vascular site while maintaining the connection between the embolizing element or elements and the carrier.

Preferably, the vaso-occlusive device is of the type that is initially in the form of an elongate, flexible, filamentous element for delivery through the microcatheter, and that assumes a three-dimensional geometry upon installation in the target vascular site. One such device is the above-described GDC (U.S. Patent No. 5,122,136- Guglielmi et al., the disclosure of which is incorporated herein by reference). Other such devices are describe in, for example, U.S. Patents Nos. 5,766,219 - Horton; 5,690,671 - McGurk et al.; and 5,911,731- Pham et al., the disclosures of which are incorporated herein by reference. Still other types of vaso-occlusive devices known in the art may also perform satisfactorily in this method.

In an alternative method described, the method comprises the steps of: (a) deploying an intravascular device to a position in a blood vessel adjacent to a target vascular site; (b) providing a vascular embolization device comprising at least one expansible embolizing element non-releasably connected to a filamentous carrier; (c) passing a microcatheter intravascularly so that the distal end of the microcatheter passes through the intravascular device into the target vascular site; (d) passing the embolization device through the microcatheter so that it emerges from the distal end of the microcatheter into the target vascular site; and (e) expanding the embolizing element or elements *in situ* substantially to fill the volume of the target vascular site while maintaining the connection between the embolizing element or elements and the carrier.

It is understood that the step of providing the embolization device may follow the step of passing the microcatheter intravascularly.

In this alternative, the intravascular device may be of the type disclosed in U.S. Patent No. 5,980,514 - Kupiecki et al., the disclosure of which is incorporated herein by reference. This intravascular device comprises a filamentous element that is introduced by a microcatheter to the juncture of an aneurysm or the like, and that then assumes the configuration of a coil adjacent the neck of the aneurysm.

In some instances, the step of passing a vaso-occlusive device or an intravascular device through the microcatheter to the target vascular site may be omitted.

The embolization bodies or elements, in the preferred embodiment, have an initial configuration in the form of small, substantially cylindrical "micropellets" of small enough outside diameter to fit within the microcatheter. The bodies are hydrophilically expansible into an expanded configuration in which they substantially conform to and fill the vascular site.

The present invention provides a number of significant advantages. Specifically, the present invention provides an effective vascular embolization device that can be deployed within a vascular site with excellent locational control, and with a lower risk of vascular rupture, tissue damage, or migration than with prior art devices. Furthermore, the embolization device effects a conformal fit within the site that promotes effective embolization, and yet its ability to be delivered to the site through a microcatheter facilitates precise and highly controllable deployment. In addition, the essentially filamentous initial configuration of the embolization device, whereby it readily conforms to the interior dimensions of the vascular site, allows it to be used effectively to embolize vascular sites having a wide variety of sizes, configurations, and (in the particular case of aneurysms) neck widths. These and other advantages will be readily appreciated from the detailed description that follows.

### BRIEF DESCRIPTION OF THE DRAWINGS

Figure 1 is an elevational view of a vascular embolization device in accordance with a preferred embodiment of the invention;
Figure 2 is a cross-sectional view taken along line 2 - 2 of Figure 1;
Figure 3 is a cross-sectional view taken along line 3 - 3 of Figure 2;
Figures 4 through 7 are semischematic views showing the steps in a method of embolizing a vascular site (specifically, an aneurysm) in accordance with one embodiment of the embolizing method aspect of the present invention;
Figure 8 is a detailed perspective view of mechanism by which the embolization device of the present invention is preferably attached to the distal end of a deployment instrument;
Figure 9 is a detailed perspective view, similar to that of Figure 8, showing the embolization device of the present invention after it has been separated from the deployment instrument;
Figures 10, 11, and 12 are semischematic views showing steps that, in addition to those illustrated in Figures 4-7, constitute a method of embolizing a vascular site in accordance with a preferred embodiment of the embolizing method aspect of the present invention; and
Figure 13 is a semischematic view showing a step in a method of embolizing a vascular site in accordance with an alternative embodiment of the embolizing method aspect of the present invention.

### DETAILED DESCRIPTION OF THE INVENTION

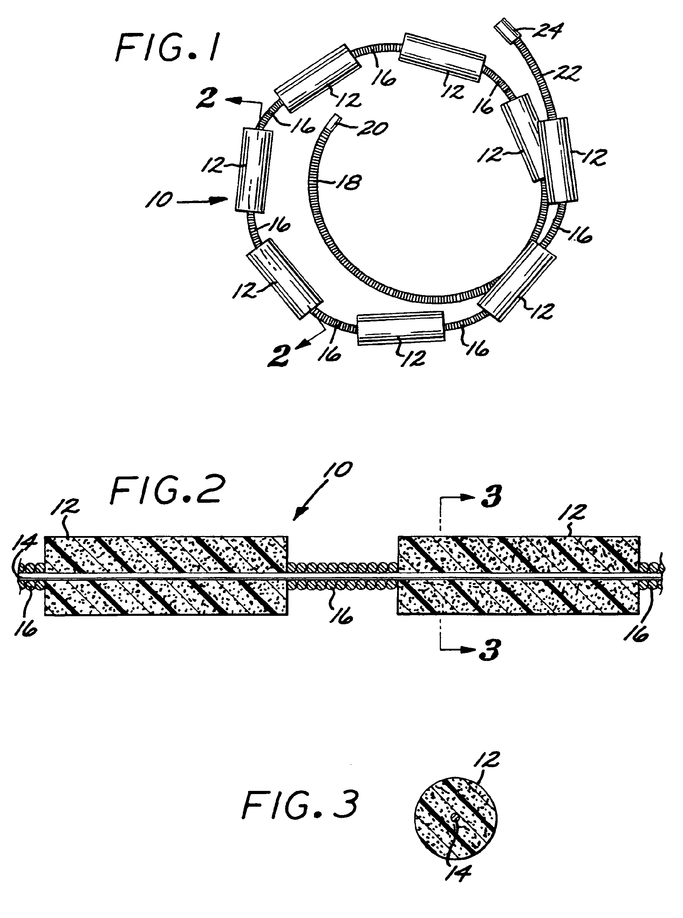

The Embolization Device. A vascular embolization device 10, in accordance with the present invention, is shown in Figures 1, 2 and 3. In the preferred embodiment, the embolization device 10 comprises a plurality of embolizing bodies, each configured as a substantially cylindrical "micropellet" 12, located at spaced intervals along a filamentous carrier 14. The number of micropellets 12 will vary, depending on the length of the carrier 14, which, turn, will depend on the size of the vascular site to be embolized. For a large vascular site, for example, eight to twelve micropellets may be used, although an even larger number may be used if necessary. In some applications (e.g., very small aneurysms), as few as one or two micropellets may be used.

Also carried on the carrier 14 is a plurality of highly flexible microcoil spacers 16, each of which is disposed between and separates a pair of micropellets 12. The carrier 14 has a distal portion on which is carried a relatively long distal microcoil segment 18 that is retained in place by a distal retention member 20. The carrier 14 has a proximal portion on which is carried a relatively long proximal microcoil segment 22. The proximal end of the device 10 is terminated by a hydrogel linkage element 24, to be described below. The spacers 16, the distal microcoil segment 18, and the proximal microcoil segment 22 are all highly flexible, and they are preferably made of platinum or platinum/tungsten wire, which has the advantages of being biocompatible and radiopaque. The micropellets 12 are non-releasably carried on the carrier 14. They may be fixed in place on the filamentous carrier 14, either may or by a suitable biocompatible, water-insoluble adhesive, or they may be simply strung loosely on the carrier 14 between successive spacers 16.

The micropellets 12 are preferably formed of a biocompatible, macroporous, hydrophilic hydrogel foam material, in particular a water-swellable foam matrix formed as a macroporous solid comprising a foam stabilizing agent and a polymer or copolymer of a free radical polymerizable hydrophilic olefin monomer cross-linked with up to about 10% by weight of a multiolefin-functional cross-linking agent. A suitable material of this type is described in U.S. Patent No. 5,570,585 - Park et al., the disclosure of which is incorporated herein by reference.

Another suitable material for the micropellets 12 is a porous hydrated polyvinyl alcohol (PVA) foam gel prepared from a polyvinyl alcohol solution in a mixed solvent consisting of water and a water-miscible organic solvent, as described, for example, in U.S. Patent No. 4,663,358 - Hyon et al., the disclosure of which is incorporated herein by reference. Other suitable PVA structures are described in U.S. Patents Nos. 5,823,198 - Jones et al. and 5,258,042 - Mehta, the disclosures of which are incorporated herein by reference. Another suitable material is a collagen foam, of the type described in U.S. Patent No. 5,456,693 - Conston et al., the disclosure of which is incorporated herein by reference. Still another suitable material is PHEMA, as discussed in the references cited above. See, e.g., Horák et al., *supra,* and Rao et al., *supra.*

The preferred foam material, as described in the above-referenced patent to Park et al., has a void ratio of at least about 90%, and its hydrophilic properties are such that it has a water content of at least about 90% when fully hydrated. In the preferred embodiment, each of the embolizing micropellets 12 has an initial diameter of not more than about 0.5 mm prior to expansion *in situ*, with an expanded diameter of at least about 3 mm. To achieve such a small size, the micropellets 12 may be compressed to the desired size from a significantly larger initial configuration. The compression is performed by squeezing or crimping the micropellets 12 in a suitable implement or fixture, and then "setting" them in the compressed configuration by heating and/or drying. Each of the micropellets 12 is swellable or expansible to many times (at least about 25 times, preferably about 70 times, and up to about 100 times) its initial (compressed) volume, primarily by the hydrophilic absorption of water molecules from an aqueous solution (e.g., resident blood plasma and/or injected saline solution), and secondarily by the filling of its pores with blood. Also, the micropellets 12 may be coated with a water-soluble coating (not shown), such as a starch, to provide a time-delayed expansion. Another alternative is to coat the micropellets 12 with a temperature-sensitive coating that disintegrates in response to normal human body temperature. See, e.g., U.S. Patents Nos. 5,120,349 - Stewart et al. and 5,129,180 - Stewart.

The foam material of the embolizing micropellet 12 may advantageously be modified, or provided with additives, to make the device 10 visible by conventional imaging techniques. For example, the foam can be impregnated with a water-insoluble radiopaque material such as barium sulfate, as described by Thanoo et al., "Radiopaque Hydrogel Microspheres", J. Microencapsulation, Vol. 6, No. 2, pp. 233-244 (1989). Alternatively, the hydrogel monomers can be copolymerized with radiopaque materials, as described in Horák et al., "New Radiopaque PolyHEMA-Based Hydrogel Particles", J. Biomedical Materials Research, Vol. 34, pp. 183-188 (1997).

The micropellets 12 may optionally include bioactive or therapeutic agents to promote thrombosis, cellular ingrowth, and/or epithelialization. See, e.g, Vacanti et al., "Tissue Engineering: The Design and Fabrication of Living Replacement Devices for Surgical Reconstruction and Transplantation," The Lancet (Vol. 354, Supplement 1), pp. 32-34 (July, 1999); Langer, "Tissue Engineering: A New Field and Its Challenges," Pharmaceutical Research, Vol. 14., No. 7, pp. 840-841 (July, 1997); Persidis, "Tissue Engineering, "Nature Biotechnology, Vol. 17, pp. 508-510 (May, 1999).

The filamentous carrier 14 is preferably a length of nickel/titanium wire, such as that marketed under the trade name "Nitinol". Wire of this alloy is highly flexible, and it has an excellent "elastic memory", whereby it can be formed into a desired shape to which it will return when it is deformed. In a preferred embodiment of the invention, the wire that forms the carrier 14 has a diameter of approximately 0.04 mm, and it is heat-treated to form a multi-looped structure that may assume a variety of three-dimensional shapes, such as a helix, a sphere, or an ovoid (as disclosed, for example, in U.S. Patent No. 5,766,219 - Horton, the disclosure of which is incorporated herein by rerefence). Preferably, the intermediate portion of the carrier 14 (i.e., the portion that includes the micropellets 12) and the proximal portion (that carries the proximal microcoil segment 22) are formed into loops having a diameter of approximately 6 mm, while the distal portion (that carries the distal microcoil segment 18) may have a somewhat greater diameter (e.g., approximately 8 -10 mm). The carrier 14 may be formed of a single wire, or it may be formed of a cable or braided structure of several ultra-thin wires.

In another embodiment, the carrier 14 may be made of a thin filament of a suitable polymer, such as a PVA, that is formed in a looped structure. The polymer may be impregnated with a radiopaque material (e.g., barium sulfate or particles of gold, tantalum, or platinum), or it may enclose a core of nickel/titanium wire. Alternatively, the carrier 14 may be constructed as a "cable" of thin polymer fibers that includes fibers of an expansile polymer, such as polyvinyl alcohol (PVA), at spaced intervals to form the micropellets 12.

Still another alternative construction for the carrier 14 is a continuous length of microcoil. In such an embodiment, the micropellets 12 would be attached at spaced intervals along the length of the carrier 14.

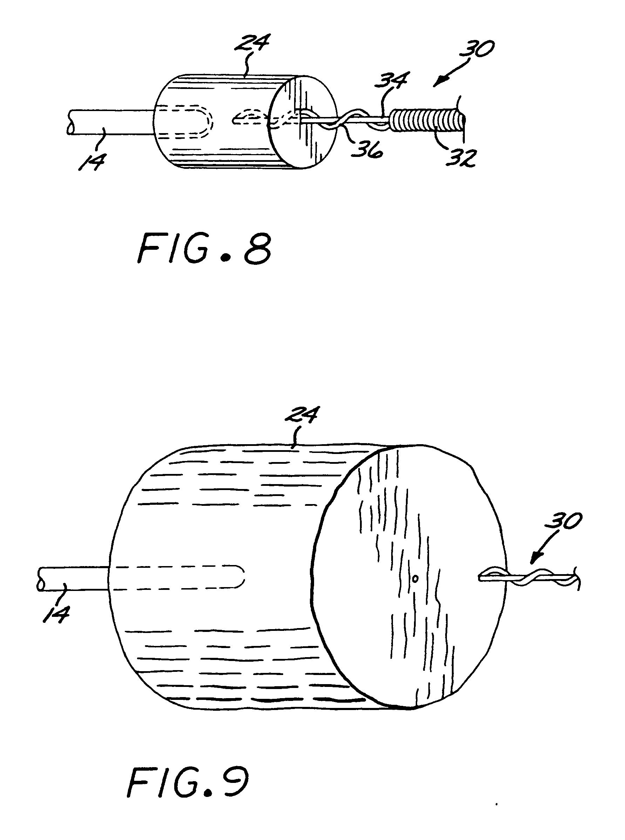

As shown in Figures 1, 8, and 9, the hydrogel linkage element 24 is advantageously made of the same material as the micropellets 12. Indeed, the most proximal of the micropellets 12 may function as the linkage element 24. The linkage element 24 is attached to the proximal end of the carrier 14 by a suitable biocompatible adhesive. The purpose of the linkage element 24 is to removably attach the device 10 to a deployment instrument 30 (Figures 8 and 9). The deployment instrument 30 comprises a length of platinum or platinum/tungsten microcoil outer portion 32 with a flexible wire core 34 of the same or a similar metal. The deployment instrument 30 has a distal portion 36 at which the microcoil outer portion 32 has coils that are more distantly-spaced (i.e., have a greater pitch).

As shown in Figure 8, the device 10 is initially attached to the deployment instrument 30 by means of the linkage element 24. Specifically, the linkage element 24 is installed, in a compressed state, so that it encompasses and engages both the proximal end of the embolization device 10 and the distal portion 36 of the deployment instrument 30. Thus, in the compressed state, the linkage element 24 binds the deployment instrument 30 and the embolization device 10 together. As shown in Figure 9, and as will be described in detail below, after the device 10 is deployed in a vascular site, the linkage element 24 expands greatly, thereby loosening its grip on the distal portion 36 of the deployment instrument 30, and thus allowing the embolization device 10 to be separated from the deployment instrument 30 by pulling the latter proximally out of and away from the linkage element 24.

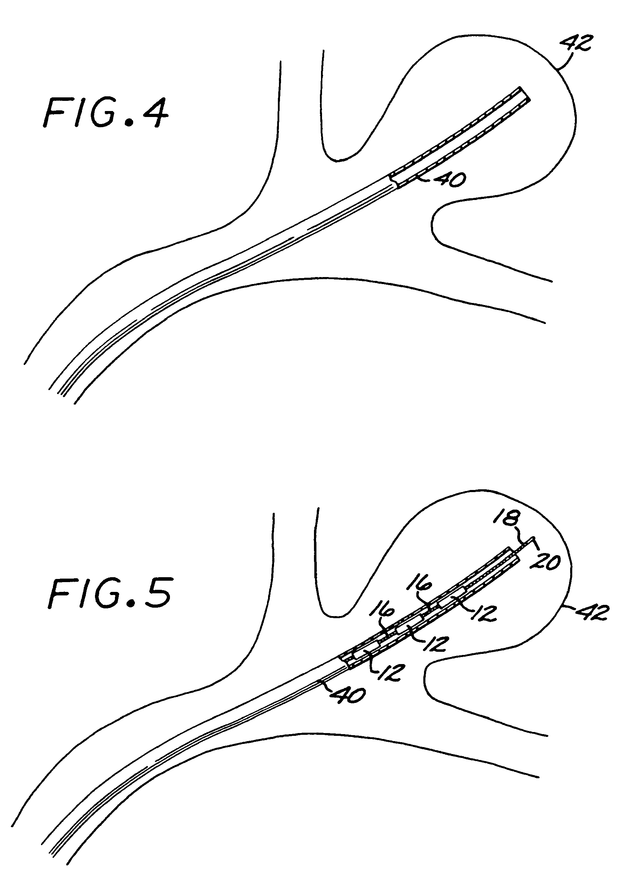

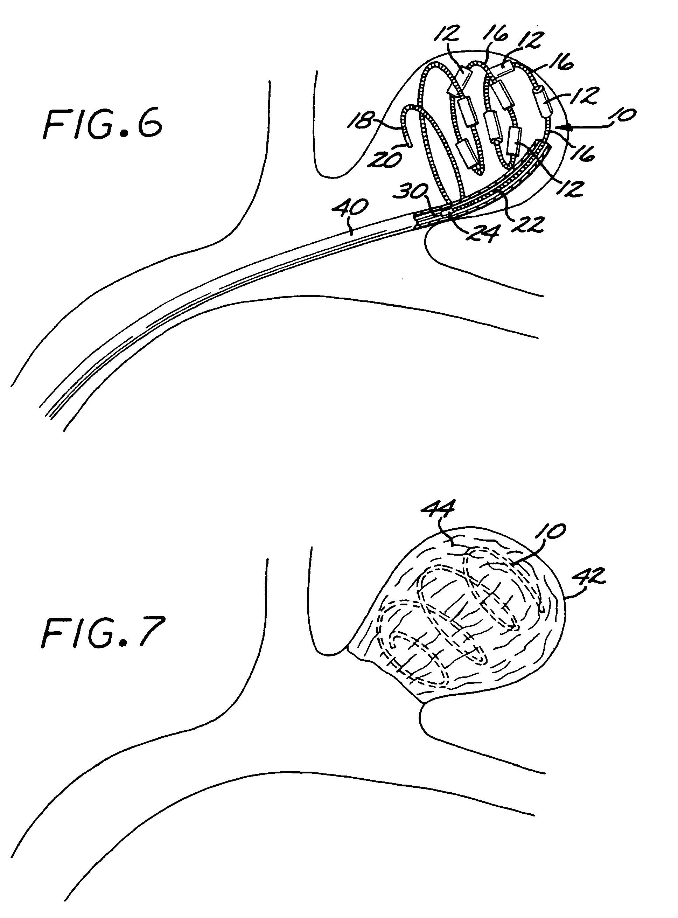

The Method for Embolizing a Vascular Site. One method of embolizing a vascular site using the embolization device 10 is illustrated in Figures 4 through 7. First, as shown in Figure 4, a microcatheter 40 is threaded intravascularly, by known methods, until its distal end is located within the targeted vascular site (here, an aneurysm 42). Briefly described, this threading operation is typically performed by first introducing a catheter guidewire (not shown) along the desired microcatheter path, and then feeding the microcatheter 40 over the catheter guidewire until the microcatheter 40 is positioned adjacent the distal aspect of the dome of the aneurysm, as shown in Figure 4. The catheter guidewire is then removed. Then, as shown in Figures 5 and 6, the embolization device 10, which is attached to the distal end of the deployment instrument 30, as described above, is passed axially through the microcatheter 40, using the deployment instrument 30 to push the device 10 through the microcatheter 40 until the device 10 is clear from the distal end of the microcatheter 40 and fully deployed within the aneurysm 42 (Figure 6), filling the aneurysm from its distal aspect. The deployment procedure is facilitated by the visualization of the embolization device 10 that is readily accomplished due to its radiopaque components, as described above.

The embolization bodies or micropellets 12, in their compressed configuration, have a maximum outside diameter that is less than the inside diameter of the microcatheter 40, so that the embolization device 10 can be passed through the microcatheter 40. The micropellets 12 are preferably compressed and "set", as described above, before the device 10 is inserted into the microcatheter 40. When inserting the device 10 into the microcatheter 40, a biocompatible, substantially non-aqueous fluid, such as polyethylene glycol, may be injected into the microcatheter 40 to prevent premature expansion of the device 10 due to hydration, and to reduce friction with the interior of the microcatheter 40.

As shown in Figure 6, when the embolization device 10 is exposed from the microcatheter 40 into the interior of the vascular site 42, the pores of the embolizing bodies or micropellets 12, and of the linkage element 24, begin to absorb aqueous fluid from the blood within the vascular site 42 to release their "set", allowing these elements to begin assuming their expanded configuration. The expansion can be enhanced and accelerated by injecting saline solution through the microcatheter 40. The expansion of the linkage element 24 allows the embolization device 10 to be separated from the deployment instrument 30, as described above, and the deployment instrument 30 can then be removed. Also, the elastic memory of the carrier 14 causes it to resume its original looped configuration once it is released from the confines of the microcatheter 40. Thus, almost immediately upon its release into the vascular site (aneurysm) 42, the embolization device begins to occupy a significant portion of the volume of the aneurysm 42.

The micropellets 12 are of a hydrophilic material, they then continue to expand *in situ* due to hydrophilic hydration of the material, as well as from the filling of their pores with blood. If the embolizing bodies 12 are of a non-hydrophilic material, their expansion is due to the latter mechanism only. In either case, the result, as shown in Figure 7, is the substantially complete filling of the interior of the aneurysm 42 with the expanded embolizing bodies or micropellets 12, whereby a substantially conformal embolizing implant 44 is formed that substantially fills the interior of the aneurysm 42. The micropellets 12, being non-releasably carried the carrier 14 and fixed in place thereon, stay on the carrier during their expansion. Thus, the chance of a micropellet separating from the carrier and migrating out of the vascular site is minimized.

It may be advantageous, prior to performing the procedural steps described above, preliminarily to visualize the aneurysm 42, by conventional means, to obtain a measurement (or at least an approximation) of its volume. Then, a device 10 of the appropriate size can be selected that would expand to fill the measured or estimated volume.

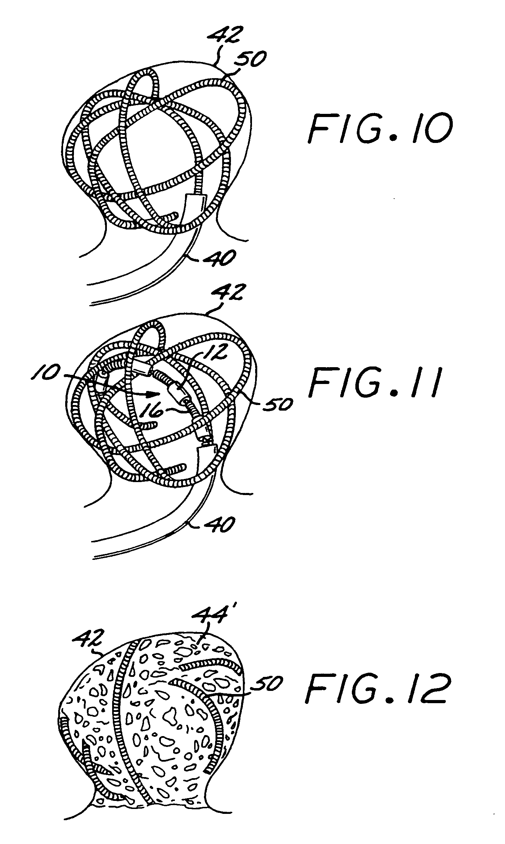

A preferred method of embolizing a target vascular site using the embolization device 10 will be understood with reference to Figures 1012, along with Figures 4-7 (discussed above). In this preferred embodiment of the method, the passing of a microcatheter 40 intravascularly until its distal end is introduced into a target vascular site (Figure 4) is followed by the step of passing a vaso-occlusive device 50 through the microcatheter 40 into the target vascular site (e.g., the aneurysm 42) so that the vaso-occlusive device 50 assumes a three-dimensional configuration that fills a portion of the interior volume of the target vascular site 42, as shown in Figure 10. The deployed vaso-occlusive device 50 forms a "cage" within the aneurysm 42 that provides a matrix for improved retention of the expansible embolizing bodies or micropellets 12 of the embolization device 10. The embolization device 10 is then passed through the microcatheter 40, as described above, and as shown in Figure 11, to enter the aneurysm 42 within the voids left by the vaso-occlusive device 50. Finally, the embolizing bodies or micropellets 12 are expanded, as described above, and as shown in Figure 12, whereby a substantially conformal embolizing implant 44' is formed that substantially fills the remaining interior volume of the aneurysm 42.

Preferably, the vaso-occlusive device 50 is of the type that is initially in the form of an elongate, flexible, filamentous element for delivery through the microcatheter, and that assumes a three-dimensional geometry (either by elastic behavior or by shape memory) upon installation in the target vascular site. Such devices are describe in, for example, U.S. Patents Nos. 5,122,136 - Guglielmi et al.; 5,766,219 - Horton; 5,690,671- McGurk et al.; and 5,911,731 - Pham et al., the disclosures of which are incorporated herein by reference. Still other types of vaso-occlusive devices known in the art may also perform satisfactorily in this method. For example, a stent-like device like that shown in U.S. Patent No. 5,980,554 - Lenker et al. may be employed Alternatively, the vaso-occlusive device 50 may be designed or installed only to enter the space near the opening or "neck" of the aneurysm. In any case, the purpose of the vaso-occlusive device 50 in this method is to present a structural framework that helps retain the embolization device 10 in place within the target vascular site.

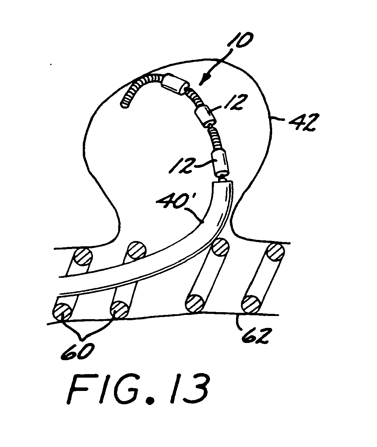

An alternative embodiment of the method of using the present invention will be understood with reference to Figure 13. In this alternative embodiment, the method includes the preliminary step of deploying an intravascular device 60 to a position in a blood vessel 62 adjacent to a target vascular site 42. A microcatheter 40' is passed intravascularly so that its distal end passes through the intravascular device 60 into the target vascular site 42. The embolization device 10 is passed through the microcatheter 40' so that it emerges from the distal end of the microcatheter 40' into the target vascular site 42, and the embolizing elements 12 are then expanded *in situ,* as described above, substantially to fill the volume of the target vascular site 42 (as shown in Figures 7 and 12).

It is understood that the step of deploying an intravascular device to a position in a blood vessel adjacent to a target vascular site would include any substeps necessary for such deployment. For example, if the intravascular device 60 is of the type disclosed in U.S. Patent No. 5,980,514 - Kupiecki et al., the deployment step would comprise the substeps of (i) passing of a microcatheter intravascularly so that its distal end is located adjacent the target vascular site; (ii) passing the intravascular device through the microcatheter until it emerges from the distal end of the microcatheter; and (iii) allowing the intravascular device to assume a three-dimensional configuration adjacent to the target vascular site. In this case, either the microcatheter used for deploying the intravascular device could be removed and then another microcatheter used to install the embolization device, or the intravascular deployment microcatheter could be repositioned for the introduction of the embolization device.

In this alternative method, the intravascular device presents an obstruction that at least partially blocks the juncture between the target vascular site and the blood vessel (e.g., the neck of an aneurysm). Thus, the intravascular device helps retain the embolization device in its proper position within the target vascular site.

Although the device 10 has been described above for use in embolizing aneurysms, other applications will readily suggest themselves. For example, it can be used to treat a wide range of vascular anomalies, such as arteriovenous malformations and arteriovenous fistulas. Certain tumors may also be treated by the embolization of vascular spaces or other soft tissue voids using the present invention.

While a preferred embodiment of the invention has been described above, a number of variations and modifications may suggest themselves to those skilled in the pertinent arts. For example, the initial shape and number of embolizing bodies 12 may be varied, as well as the length of the carrier 14. Furthermore, other mechanisms may be found for removably attaching the embolization device 10 to the deployment wire. One such alternative attachment mechanism may be a transition polymer joint that loosens when heated by contact with blood or by a low-level electric current. These and other variations and modifications are considered within the scope of the invention, as described in the claims that follow.

## Claims

1. A device (10) for embolizing a vascular site comprising:
one or more expansible, hydrophilic embolizing elements (12) non-releasably carried on a filamentous, elastic memory carrier (14) at spaced intervals along the length of the carrier (14), wherein said one or more embolizing elements (12) are cylindrical micropellets.

2. The device of Claim 1, wherein the embolizing element (12) is formed of a hydrophilic hydrogel foam material.

3. The device of Claim 2 wherein the foam material includes a water-swellable foam matrix formed as a macroporous solid comprising a foam stabilizing agent and a polymer or copolymer of a free radical polymerizable hydrophilic olefin monomer cross-linked with up to about 10% by weight of a multiolefin-functional cross-linking agent

4. The device of Claim 1, wherein the embolizing element (12) is formed of a material selected from the group consisting of polyvinyl alcohol foam, collagen foam, and poly (2-hydroxyethyl methacrylate).

5. The device of Claim 1, wherein the embolizing element (12) has an initial diameter of not more than about 0.5 mm and is expansible to a diameter of at least about 3.0 mm.

6. The device of Claim 1, wherein the embolizing element (12) has a predetermined initial volume and is expansible to an expanded volume that is at least about 25 times its initial volume.

7. The device of Claim 1, wherein the embolizing element (12) is a first embolizing element, the device further comprising at least a second expansible embolizing element (12) mechanically connected to the carrier (14) at a fixed location thereon spaced from the first expansible embolizing element (12).

8. The device of Claim 7, further comprising a microcoil spacer (16) located on the carrier (14) between the first and second expansible embolizing elements (12).

9. The device of Claim 1, wherein the carrier (14) includes a thin, flexible metal wire (14) formed into a multi-looped configuration.

10. The device of Claim 9, wherein the wire (14) is made of an alloy of nickel and titanium that exhibits good elastic memory properties.

11. The device of Claim 1, wherein the carrier (14) includes a thin filament (14) of polymer formed into a multi-looped configuration.

12. The device of Claim 1, wherein the carrier (14) is formed of a flexible material having an elastic memory and is initially configured in a multi-loop configuration, and wherein a plurality of the expansible embolizing elements (12) are located at spaced intervals along the length of the carrier (14).

13. The device of Claim 12, wherein the carrier (14) has an intermediate portion on which the expansible embolizing elements (12) are located, a proximal portion (22), and a distal portion (18).

14. The device of Claim 13, wherein the intermediate portion is formed into at least one loop of approximately a first diameter, the proximal portion (22) is formed into at least one loop of approximately the first diameter, and the distal portion (18) is formed into at least one loop of approximately a second diameter that is greater than the first diameter.

15. The device of Claim 13, further comprising an expansible linkage element (24) on the proximal portion (22).

16. The device of Claim 15, wherein the linkage element (24) is formed of the same material as are the embolizing elements (12)_{.}

17. The device of Claim 12, wherein the embolizing elements (12) are formed of a hydrophilic hydrogel foam material.

18. The device of Claim 17, wherein the foam material includes a water-swellable foam matrix formed as a macroporous solid comprising a foam stabilizing agent and a polymer or copolymer of a free radical polymerizable hydrophilic olefin monomer cross-linked with up to about 10% by weight of a multiolefin-functional cross-linking agent.

19. The device of Claim 12, wherein the embolizing elements (12) are formed of a material selected from the group consisting of polyvinyl alcohol foam, collagen foam, and poly (2-hydroxyethyl methacrytate).

20. The device of Claim 12, wherein the embolizing elements (12) have an initial diameter of not more than about 0.5 mm and are expansible to a diameter of at least about 3.0 mm.

21. The device of Claim 12, wherein the embolizing elements (12) have a predetermined initial volume and are expansible to an expanded volume that is at least about 25 times their initial volume.

## Patentansprüche

1. Eine Vorrichtung (10) zur Embolisation einer Gefäßstelle, umfassend:
ein oder mehrere ausdehnbare, hydrophile emboliserende Elemente (12), die auf einem faserförmigen, elastischen Gedächtnisträger in Abständen entlang der Länge des Trägers (14) lösbar getragen werden, wobei das eine oder mehrere embolisierende Elemente (12) zylindrische Mikropellets sind.

2. Vorrichtung nach Anspruch 1, wobei das embolisierende Element (12) aus einem hydrophilen Schaummaterial gebildet ist.

3. Vorrichtung nach Anspruch 2, wobei das Schaummaterial eine wasserquellbare Schaummatrix mit der Form eines makroporösen Feststoffes umfasst, und umfassend ein schaumstabilisierendes Mittel und ein Polymer oder Copolymer eines frei radikalen polymerisierbaren hydrophilen Olefinmononers, das mit bis zu ungefähr 10 Gew.% eines multiolefin-funktionellen Querverbindungsmittels querverbunden ist.

4. Vorrichtung nach Anspruch 1, wobei das embolisierende Element (12) aus einem aus der Gruppe bestehend aus Polyvinylalkoholschaum, Kollagenschaum und Poly-(2-hydroxyethylmethacrylat) ausgewählten Material gebildet ist.

5. Vorrichtung nach Anspruch 1, wobei das embolisierende Element (12) einen Ausgangsdurchmesser von höchstens ungefähr 0,5 mm aufweist und auf einen Durchmesser von mindestens ungefähr 3,0 mm ausdehnbar ist.

6. Vorrichtung nach Anspruch 1, wobei das embolisierende Element (12) ein vorausbestimmtes Anfangsvolumen aufweist und auf ein ausgedehntes Volumen ausdehnbar ist, das mindestens ungefähr 25 mal seines Anfangsvolumens beträgt.

7. Vorrichtung nach Anspruch 1, wobei das embolisierende Element (12) ein erstes embolisierendes Element ist, wobei die Vorrichtung ferner mindestens ein zweites embolisierendes Element (12) umfasst, das im Abstand vom ersten emboliserenden Element (12) an einer festgesetzten Stelle mit dem Träger (14) mechanisch verbunden ist.

8. Vorrichtung nach Anspruch 7, wobei sie ferner einen Mikrospulenabstandshalter (14) umfasst, der auf dem Träger zwischen dem ersten und zweiten ausdehnbaren embolisierenden Element (12) angeordnet ist.

9. Vorrichtung nach Anspruch 1, wobei der Träger (14) einen dünnen, flexiblen Metalldraht (14) umfasst, der zu einer Mehrschleifenkonfiguration gebildet ist.

10. Vorrichtung nach Anspruch 9, wobei der Draht (14) aus einer Legierung aus Nickel und Titanium hergestellt ist, die gute elastische Gedächtniseigenschaften aufweist.

11. Vorrichtung nach Anspruch 1, wobei der Träger (14) einen dünnen Faden (14) aus Polymer umfasst, der zu einer Mehrschleifenkonfiguration gebildet ist.

12. Vorrichtung nach Anspruch 1, wobei der Träger (14) aus einem flexiblen Material mit einem elastischen Gedächtnis gebildet ist und anfangs in einer Mehrschleifenkonfiguration konfiguriert ist, und wobei eine Vielzahl der ausdehnbaren embolisierenden Elemente (12) in Abständen entlang der Länge des Trägers (14) angeordnet ist.

13. Vorrichtung nach Anspruch 12, wobei der Träger (14) einen Zwischenteil, auf welchem die ausdehnbaren embolisierenden Elemente (12) angeordnet sind, einen proximalen Teil (22) und einen distalen Teil (18) aufweist.

14. Vorrichtung nach Anspruch 13, wobei der Zwischenteil zu mindestens einer Schleife von ungefähr einem ersten Durchmesser gebildet ist, der proximale Teil (22) zu mindestens einer Schleife von ungefähr dem ersten Durchmesser gebildet ist, und der distale Teil (18) zu mindestens einer Schleife von ungefähr einem zweiten Durchmesser gebildet ist, der größer als der ersten Durchmesser ist.

15. Vorrichtung nach Anspruch 13, ferner umfassend ein ausdehnbares Verbindungsglied (24) auf dem proximalen Teil (22).

16. Vorrichtung nach Anspruch 15, wobei das Verbindungsglied (24) aus dem gleichen Material wie die embolisierenden Elemente (12) gebildet ist.

17. Vorrichtung nach Anspruch 12, wobei die embolisierenden Elemente (12) aus einem hydrophilen Hydrogelschaummaterial gebildet ist.

18. Vorrichtung nach Anspruch 17, wobei das Schaummaterial eine wasserquellbare Schaummatrix mit der Form eines makroporösen Feststoffes umfasst, und umfassend ein schaumstabilisierendes Mittel und ein Polymer oder Copolymer eines frei radikalen polymerisierbaren hydrophilen Olefinmononers, das mit bis zu ungefähr 10 Gew.-% eines multiolefin-funktionellen Querverbindungsmittels querverbunden ist.

19. Vorrichtung nach Anspruch 12, wobei die embolisierenden Elemente (12) aus einem aus der Gruppe bestehend aus Polyvinylalkoholschaum, Kollagenschaum und Poly-(2-hydroxyethylmethacrylat) ausgewählten Material gebildet sind.

20. Vorrichtung nach Anspruch 12, wobei die embolisierenden Elemente (12) einen Ausgangsdurchmesser von höchstens ungefähr 0,5 mm aufweisen und auf einen Durchmesser von mindestens ungefähr 3,0 mm ausdehnbar sind.

21. Vorrichtung nach Anspruch 12, wobei die embolisierenden Elemente (12) ein vorausbestimmtes Anfangsvolumen aufweisen und auf ein ausgedehntes Volumen ausdehnbar sind, das mindestens ungefähr 25 mal ihres Anfangsvolumens beträgt.

## Revendications

1. Dispositif (10) pour l'embolisation d'un site vasculaire comprenant:
au moins un élément d'embolisation expansible et hydrophile (12) porté de manière non-dégagéable sur un transporteur (14) de mémoire élastique et filamenteux à des intervalles espacés le long de la longueur du transporteur (14), où lesdits un ou plusieurs éléments d'embolisation (12) sont des micropellets cylindriques.

2. Le dispositif de la revendication 1, où l'élément d'embolisation (12) est formé d'une matière mousseuse d'hydrogel hydrophile.

3. Le dispositif de la revendication 2, où la matière mousseuse comprend une matrice mousseuse gonflable à l'eau formée comme un solide macroporeux comprennant un agent de stabilisation mousseuse et un polymère ou copolymère d'un monomère oléfiant hydrophile de polymèrisation à radicaux libres réticulé avec jusqu'à peu près 10% du poids d'un agent réticulant multi-oléfine functionelle.

4. Le dispositif de la revendication 1, où l'élément d'embolisation (12) est formé d'une matière selectionnée du groupe consistant de mousse d'alcool polyvinylique, de mousse collagène, et de poly (2-méthacrylate d'hydroxyéthyle).

5. Le dispositif de la revendication 1, où l'élément d'embolisation (12) a un diamètre initial qui n'excède pas environ 0.5 mm et qui est expansible à un diamètre d'au moins environ 3.0 mm.

6. Le dispositif de la revendication 1, où l'élément d'embolisation (12) a un volume initial prédéterminé et étant expansible à un volume étendu qui est au moins d'environ 25 fois son volume initial.

7. Le dispositif de la revendication 1, où l'élément d'embolisation (12) est un premier élément d'embolisation, le dispositif comprenant en outre au moins un deuxième élément embolisation expansible (12) étant lié mécaniquement au transporteur (14) à une localisation fixée la dessus espacé du premier élément d'embolisation expansible (12).

8. Le dispositif de la revendication 7, comprenant en outre une microbobine d'espacement (16) localisée sur le transporteur (14) entre le premier et le deuxième éléments d'embolisation expansibles (12).

9. Le dispositif de la revendication 1, où le transporteur (14) comprend un fil métallique mince et flexible (14) formé en une configuration multi-boucles.

10. Le dispositif de la revendication 9, où le fil (14) est fait d'un alliage de nickel et de titane faisant preuve de bonnes propriétés de mémoire élastique.

11. Le dispositif de la revendication 1, où le transporteur (14) comprend un fil mince (14) de polymère formé en une configuration multi-boucles.

12. Le dispositif de la revendication 1, où le transporteur (14) est formé d'une matière flexible ayant une mémoire élastique et étant initiallement configuré en une configuration multi-boucles et où une pluralité des éléments d'embolisation expansibles (12) est localisée à des intervalles espacés le long de la longueur du transporteur (14).

13. Le dispositif de la revendication 12, où le transporteur (14) a une portion intermédiaire sur laquelle les éléments d'embolisation expansibles (12) sont localisés, une portion proximale (22) et une portion distale (18).

14. Le dispositif de la revendication 18, où la portion intermédiaire est formée en au moins une boucle d'environ un premier diamètre, la portion proximale (22) étant formée en au moins une boucle d'environ le premier diamètre, et la portion distale (18) est formée en au moins une boucle d'environ un deuxième diamètre qui est plus grand que le premier diamètre.

15. Le dispositif de la revendication 13, comprenant en outre un élément de liaison (24) expansible sur la portion proximale (22).

16. Le dispositif de la revendication 15, où l'élément de liaison (24) est formé de la même matière que les éléments d'embolisation (12).

17. Le dispositif de la revendication 12, où les éléments d'embolisation (12) sont formés d'une matière mouseusse d'hydrogel hydrophile.

18. Le dispositif de la revendication 17, où la matière mousseuse comprend une matrice mousseuse gonflable à l'eau formée comme un solide macroporeux comprennant un agent de stabilisation mousseuse et un polymère ou copolymère d'un monomère oléfiant hydrophile de polymérisation à radicaux libres réticulé avec jusqu'à peu près 10% du poids d'un agent réticulant multi-oléfine functionelle.

19. Le dispositif de la revendication 12, où l'élément d'embolisation (12) est formé d'une matière selectionnée du groupe consistant de mousse d'alcool polyvinylique, de mousse collagène, et de poly (2-méthacrylate d'hydroxyéthyle).

20. Le dispositif de la revendication 12, où l'élément d'embolisation (12) a un diamètre initial qui n'excède pas environ 0.5 mm et qui est expansible à un diamètre d'au moins environ 3.0 mm.

21. Le dispositif de la revendication 12, où l'élément d'embolisation (12) a un volume initial prédéterminé et étant expansible à un volume étendu qui est au moins d'environ 25 fois son volume initial.
